Europäisches Patentamt

(19) European Patent Office

Office européen des brevets

(11) Veröffentlichungsnummer: **0 238 860**

**B1**

(12) **EUROPÄISCHE PATENTSCHRIFT**

(45) Veröffentlichungstag der Patentschrift: **14.11.90**

(21) Anmeldenummer: **87102492.3**

(22) Anmeldetag: **12.01.85**

(60) Veröffentlichungsnummer der früheren Anmeldung nach Art. 76 EPÜ: **0 159 462**

(51) Int. Cl.⁵: **A 61 F 2/36**

(54) Femurale Totalendoprothese für ein Hüftgelenk.

(30) Priorität: **23.03.84 DE 3410652**
**28.04.84 DE 3415934**

(43) Veröffentlichungstag der Anmeldung:
**30.09.87 Patentblatt 87/40**

(45) Bekanntmachung des Hinweises auf die Patenterteilung:
**14.11.90 Patentblatt 90/46**

(84) Benannte Vertragsstaaten:
**AT BE CH DE FR GB IT LI NL SE**

(56) Entgegenhaltungen:
**CH-A- 552 383**
**DE-U-8 127 991**
**FR-A-2 528 307**
**US-A-2 719 522**

(73) Patentinhaber: **orthoplant Endoprothetik GmbH**
**Leerkämpe 12**
**D-2800 Bremen 66 (DE)**

(72) Erfinder: **Schelhas, Klaus-Dieter**
**Leerkämpe 12**
**D-2800 Bremen 66 (DE)**

(74) Vertreter: **Eisenführ, Speiser & Strasse**
**Martinistrasse 24**
**D-2800 Bremen 1 (DE)**

Courier Press, Leamington Spa, England.

**Beschreibung**

Die Erfindung betrifft eine femurale Totalendoprothese für ein Hüftgelenk, mit einem Femurkopf an einem Schaft, dessen Kern unterhalb des Femurkopfes einen in senkrechter Richtung zu einer medial/Lateralen Ebene abgeflachten, gekrümmten oberen Abschnitt, und einen sich konisch verjüngenden unteren Abschnitt aufweist, und mit einem an der lateralen Stirnseite des oberen Schaftabschnittes angeordneten, sich in einer medial/lateralen Ebene erstreckenden, nasenförmigen Ansatz.

Derartige femurale Totalendoprothesen für Hüftgelenke sind bekannt, bei denen an der lateralen Stirnseite des oberen Schaftabschnitts ein nasenförmiger Ansatz angeordnet ist, welcher als schmaler Steg ausgebildet ist und zum Ansetzen von chirurgischen Instrumenten zum Implantieren bzw. zur Reoperation der Prothese dient. Die Totalendoprothese kommt in diesem Bereich des Schaftkernes nicht oder nur an der Außenkante des stegförmigen Ansatzes mit dem Knochengewebe in Berührung, so daß dieser Bereich zur Festigkeit des Prothesen/Knochenverbundes wegen der Form dieses bekannten Ansatzes wenig beiträgt.

Die Aufgabe der Erfindung ist es demgegenüber, die femurale Totalendoprothese der eingangs genannten Art derart weiterzubilden, daß auch der obere laterale Stirnseitenbereich der Prothese zur Fixation in dem Femurknochen beiträgt.

Diese Aufgabe wird bei einer femuralen Totalendoprothese der eingangs genannten Art erfindungsgemäß dadurch gelöst, daß der nasenförmige Ansatz an seinem dem Schaft abgekehrten lateralen Endabschnitt mit einem quer verlaufenden Flansch versehen ist.

Bevorzugt steht der quer zum nasenförmigen Ansatz verlaufende Flansch beidseitig symmetrisch über den Ansatz über, wobei es sich als zweckmäßig erwiesen hat, wenn sich der Flansch—in Anpassung an die Form des mehr oder weniger parallel zu ihm verlaufenden oberen Schaftabschnitts der Prothese nach unten hin verjüngt.

Als besonders zweckmäßig hat es sich herausgestellt, wenn der Flansch wenigstens an seiner Außenseite zum Schaft hin gekrümmt ausgebildet ist und mit der lateralen Oberfläche des unteren Abschnitts des Schaftkerns in Längsrichtung fluchtet. Dadurch ist die zusätzliche Abstützung der Prothese mittels des Flansches am Trochantermassiv des Femur besonders günstig und großflächig, wodurch die Verankerung der Prothese im Knochen, insbesondere auch die Primärfixation nach der Implantation wesentlich verbessert wird.

Besonders bevorzugt ist der nasenförmige Ansatz und/oder der Flansch mit Durchgangsöffnungen versehen, in die das Knockengewebe nach der Implantation der Prothese einwachsen kann, um auf diese Weise die Verankerung am Trochantermassiv besonders innig und wirksam zu machen.

Bevorzugte Ausgestaltungen der vorliegenden Erfindung sind in den Unteransprüchen beschrieben.

Die Erfindung ist nachstehend an einem Ausführungsbeispiel unter Bezugnahme auf eine Zeichnung weiter erläutert. Es zeigen:

Fig. 1 eine Seitenansicht einer erfindungsgemäßen Hüftgelenk-Totalendoprothese ohne Femurkopf;

Fig. 2 eine Ansicht der Prothese gemäß Fig. 1 in Richtung des Pfeiles II in Fig. 1 gesehen;

Fig. 3 eine Teilansicht der Prothese gemäß den Fig. 1 und 2 in Richtung des Pfeiles III in Fig. 1 gesehen;

Fig. 4 einen Querschnitt durch den Schaft der Prothese gemäß den Fig. 1 bis 3 in Richtung der Schnittlinie IV—IV gesehen;

Fig. 5 einen etwas weiter obenliegenden Querschnitt durch den Prothesenschaft mit einer anders ausgestalteten Profilierung.

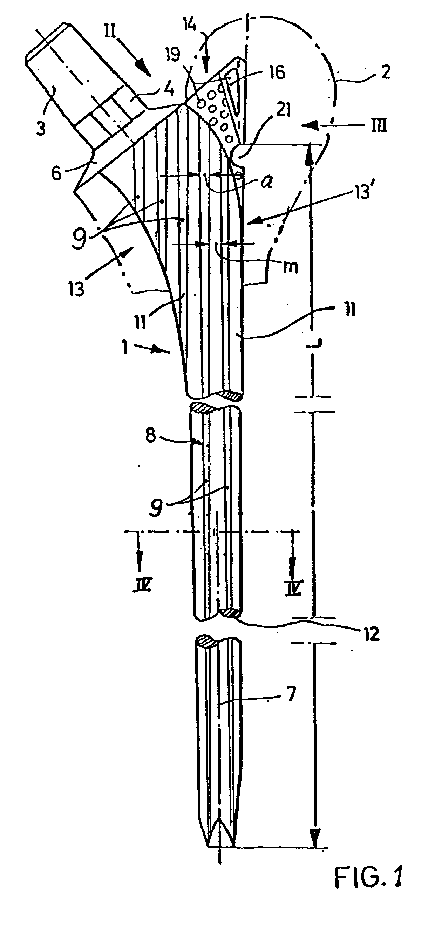

Die Fig. 1 bis 4 zeigen eine Totalendoprothese für ein Hüftgelenk mit einem im Markkanal des Femurknochens zu verankernden Schaft 1. Der Femurknochen ist im wesentlichen nicht dargestellt und lediglich in seinem oberen Endabschnitt—nach Resektion des natürlichen Femurkopfes unter einem Winkel von 50°—mit einer strichpunktierten Linie 2 angedeutet.

Der Schaft 1 ist an seinem oberen Endabschnitt mit einem kugelförmigen Femurkopf versehen, der in der Zeichnung nicht dargestellt ist. Der nicht dargestellte Femurkopf wird auf einen konischen Zapfen 3 aufgesteckt, um auf diese Weise unterschiedliche Kugelkopfgrößen verwirklichen zu können. Unterhalb des Zapfens 3 befindet sich ein integral mit diesem ausgebildeter Mehrkant 4, an dem die Prothese mit einem entsprechenden Schlüssel gehalten bzw. gedreht werden kann, und unterhalb des Mehrkant 4 befindet sich ein flanschförmiger Kragen 6, wie er beispielsweise in der DE—OS 32 47 726 dargestellt und beschrieben ist.

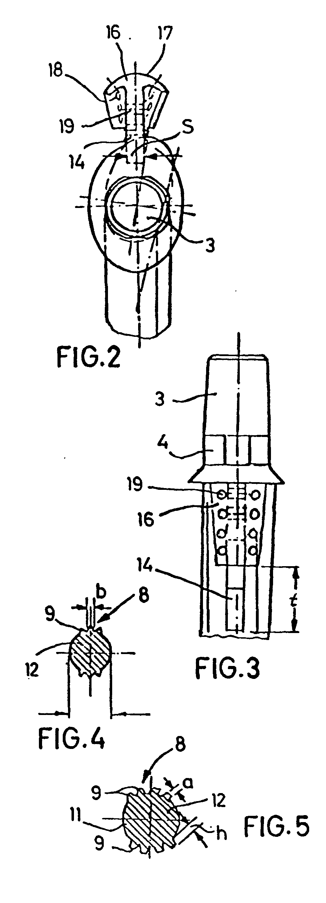

Der unterhalb des Femurkopfes bzw. des Kragens 6 liegende obere Abschnitt des Schaftkerns ist in einer senkrecht zu einer medial/lateralen Ebene liegenden Richtung abgeflacht und in seinem oberen Abschnitt in Medialrichtung einwärts gekrümmt, wobei dieser gekrümmte Abschnitt sich verjüngend in den unteren Schaftabschnitt übergeht, der sich von oben nach unten weiterhin konisch verjüngt und einen im wesentlichen kreisförmigen Querschnitt aufweist (s.z.B. Fig. 4, 5).

Die Oberfläche des Schaftes 1 ist zu einem großen Teil mit einer sich im wesentlichen in Längsrichtung gemäß der Achse 7 des unteren Abschnittes erstreckenden Profilierung 8 versehen, die aus rippenförmigen Ansätzen 9 besteht, welche sich von der glatten Basisumfangsfläche 11 des Schaftkernes 12 aus nach außen erstrecken und integral mit dem Schaftkern 12 ausgebildet sind. In der Ausgestaltung gemäß Fig. 4 sind die rippenförmigen Ansätze 9 dreiecksförmig ausgebildet mit leicht abgerundeten Scheiteln, während sie bei der Ausgestaltung gemäß Fig. 5 trapezförmig ausgebildet sind.

Die rippenförmigen Ansätze 9 verjüngen sich vom oberen Ende des Schaftes 1 zu dessen unterem, diestalen Ende hin, indem sowohl die Höhe h als auch die Breite a nach unten hin abnehmen. Die Breite a, die an dem Fuß der Ansätze 9, unmittelbar am Schaftkern 12 gemessen wird, und die Höhe h reduzieren sich dabei nach unten hin bis auf etwa die Hälfte oder ein Drittel ihres Anfangswertes. So nimmt zum Beispiel die Breite a von etwa 3,5 auf 1,0 mm, und die Höhe h von zum Beispiel 1,5 auf 0,5 mm ab.

Die rippenförmigen Ansätzte 9 besitzen—von Fuß zu Fuß—einen kleinsten gegenseitigen Abstand m von zum Beispiel 2,5 mm, der aufgrund der abnehmenden Breite der rippenförmigen Ansätze zum unteren Schaftende hin zunimmt, während die Scheitel der Ansätze 9 über die gesamte Länge der Ansätze einen konstanten Abstand voneinander besitzen und parallel zur Längsachse 7 des Schaftkernes verlaufen.

Diese Ausgestaltung der rippenförmigen Ansätze 9 ist aus verschiedenen Gründen besonders zweckmäßig. Zum einen führt sie beim Implantieren zu einer Art Verkeilung der rippenförmigen Ansätze 9 im natürlichen Knochengewebe, derart, daß auch das Knochengewebe zwischen den Ansätzen beim Einsetzen der Prothese zunehmend verdichtet und damit verfestigt wird. Zum anderen läßt sich bei einer erforderlichen Reoperation eine solche Prothese trotz der mit ihr im implantierten Zustand erreichten optimalen Rotationssicherung leicht wieder herausziehen, wenn die Prothese erste einmal in Längsrichtung um eine verhältnismäßig geringfügige axiale Länge gelockert worden ist. Außerdem schneiden sich die Ansätze 9 im Knochengewebe ihre Sitzkanäle beim Einsetzen der Prothese selbst und belasten dabei das Knochengewebe nur ganz allmählich zunehmend stärker.

Die Länge L des Schaftes beträgt bei dem dargestellten Ausführungsbeispiel 360 mm. Die Länge der rippenförmigen Ansätze 9 erstreckt sich lediglich von oben über die gesamte Länge des Schaftes. An den beiden einander gegenüberliegenden Stirnseiten 13 und 13' des Schaftes 1 sind keine rippenförmigen Ansätze 9 vorhanden.

An der lateralen Stirnseite des oberen Schaftabschnittes befindet sich ein nasenförmiger Ansatz 14, dessen Dicke s kleiner ist als die Dicke des oberen Schaftabschnittes. Der nasenförmige Ansatz 14 weist an seinem dem Schaft 1 abgekehrten lateralen Ende einen quer verlaufenden Flansch 16 auf, der symmetrisch zu dem nasenförmigen Ansatz 14 angeordnet ist, sich also beiderseits des Ansatzes 14 über diesen hinaus erstreckt. Der Flansch 16 verjüngt sich von oben nach unten, wie aus Fig. 3 erkennbar ist, und ist sowohl an seiner Außenseite 17 als auch an seiner Innenseite 18 zum Schaft 1 hin gekrümmt ausgebildet. Der Flansch 16 ist ebenso wie der nasenförmige Ansatz 14 mit Durchgangsöffnungen 19 versehen, die einen Durchmesser von 2,5 mm aufweisen. Der Flansch 16 endet mit einem Abstand t zum unteren Ende des nasenförmigen Ansatzes 14. Letzterer ist unterhalb des Flansches 16 mit einer halbkreisförmigen Ausnehmung 21 versehen.

Wie bereits weiter oben angedeutet worden ist, wird zur Implantation einer erfindungsgemäßen Hüftgelenkprothese zunächst der natürliche Femurkopf unter einem Winkel von 50° resiziert und der Trochanter mit einer nutförmigen Ausnehmung versehen, so daß sie von oben her in den Femurknochen 2 eingeführt werden kann, wobei sie beim Einführen mittels eines entsprechenden Schlüssels am Mehrkant 4 um seine Achse 7 gedreht werden kann. Dabei kommen die rippenförmigen Ansätze 9 ebenso wie der aus dem nasenförmigen Ansatz 14 und dessen Flansch 16 bestehende T-förmige "Flügel" sogleich bei der Implantation mit dem Knochengewebe in formschlüssigen Eingriff, so daß sich unverzüglich selbst bei der zementfreien Implantation eine hohe Drehsicherheit ergibt. Selbst wenn im Verlaufe der Zeit Gewebe in die Nuten der Profilierung 8 sowie die Durchgangsöffnungen 19 einwächst, ist erforderlichenfalls eine Entfernung des Schaftes aus dem Knochengewebe in Richtung der Achse 7 öhne beachtliche Beschädigung des Gewebes möglich, da sich—bis auf ein Einwachsen in den Durchgangsöffnungen 19—an der Profilierung 8 keine quer oder schräg zur Längsachse 7 verlaufenden Hinterschneidungen befinden. Der T-förmige Abschnitt 14, 16 bezieht das Trochantermassiv festigkeitsmäßig praktisch voll ein und sorgt demgemäß für eine entsprechende Entlastung des Prothesenschaftes 1 bei dessen Belastung, da die gegenüber dem Adam-'schen Bogen bei Belastung stets herrschenden Zugkräfte z.T. von dem natürlichen Gewebe selbst übertragen werden können. Trotz dieser zahlreichen erheblichen Vorteile, die sich insbesondere günstig auswirken, wenn die Prothese in ihrer übrigen Ausgestaltung im wesentlichen der Prothese gemäß der DE—OS 32 47 726 entspricht, ergibt sich aufgrund der physiologischen Paßform, d.h. einer optimalen Anpassung an die natürlichen Gegebenheiten, eine hervorragende Abstützung im Gewebe.

Der Flansch 16 des nasenförmigen Ansatzes 14 ist mit zwei symmetrisch angeordneten, fensterförmigen Durchgangsöffnungen 19 versehen.

**Patentansprüche**

1. Femurale Totalendoprothese für ein Hüftgelenk, mit einem Femurkopf an einem Schaft (1), dessen Kern (12) unterhalb des Femurkopfes einen in senkrechter Richtung zu einer medial/lateralen Ebene abgeflachten, gekrümmten oberen Abschnitt, und einen sich konisch verjüngenden unteren Abschnitt aufweist, mit einem an der lateralen Stirnseite des oberen Schaftabschnittes angeordneten, sich in einer medial/lateralen Ebene erstreckenden, nasenförmigen Ansatz (14), dadurch gekennzeichnet, daß der nasenförmige Ansatz (14) an seinem dem Schaft (1) angekehrten lateralen Endabschnitt mit einem quer verlaufenden Flansch (16) versehen ist.

2. Femurale Totalendoprothese nach Anspruch 1, dadurch gekennzeichnet, daß sich der Flansch (16) nach unten hin verjüngt.

3. Femurale Totalendoprothese nach Anspruch 1 oder 2, dadurch gekennzeichnet, daß der Flansch (16) wenigstens an seiner Außenseite (17) zum Schaft (1) hin gekrümmt ausgebildet ist.

4. Femurale Totalendoprothese nach Anspruch 3, dadurch gekennzeichnet, daß der gekrümmte Flansch (16) mit der lateralen Oberfläche des unteren Schaftabschnitts des Schaftkerns (12) fluchtet.

5. Femurale Totalendoprothese nach einem der vorstehenden Ansprüche, dadurch gekennzeichnet, daß der Flansch (16) beidseitig symmetrisch zu dem Ansatz (14) ausgebildet ist.

6. Femurale Totalendoprothese nach einem der vorstehenden Ansprüche, dadurch gekennzeichnet, daß der nasenförmige Ansatz (14) und/oder der Flansch (16) mit Durchgangsöffnungen (19) versehen ist.

## Revendications

1. Endoprothèse totale fémorale pour une articulation de la hanche, comprenant une tête de fémure située sur une tige (1), dont le noyau (12) présente, en dessous de la tête de fémur, suivant la direction perpendiculaire à un plan latéral médian, une section supérieure aplatie et courbée et une section inférieure se rétrécissant de manière conique, et comprenant un épaulement (14) en forme de nez, qui est agencé sur le côté frontal latéral de la section de tige supérieure et qui s'étend dans un plan latéral médian, caractérisée en ce que l'épaulement en forme de nez (14) est pourvu, sur sa section d'extrémité latérale opposée à la tige (1), d'une patte (16) s'étendant transversalement.

2. Endoprothèse total fémorale suivant la revendication 1, caractérisée en ce que la patte (16) se rétrécit vers le bas.

3. Endoprothèse totale fémorale suivant la revendication 1 ou 2, caractérisée en ce que la patte (16) est réalisée, au moins sur son côté extérieur (17), de manière courbée vers la tige (1).

4. Endoprothèse totale fémorale suivant la revendication 3, caractérisée en ce que la patte courbée (16) est en alignement avec la surface latérale de la section de tige inférieure du noyau de la tige (12).

5. Endoprothèse totale fémorale suivant l'une quelconque des revendications précédentes, caractérisée en ce que la patte (16) est réalisée des deux côtés de manière symétrique par rapport à l'épaulement (14).

6. Endoprothèse totale fémorale suivant l'une quelconque des revendications précédentes, caractérisée en ce que l'épaulement en forme de nez (14) et/ou la patte (16) sont pourvus d'ouvertures de passage (19).

## Claims

1. Femural complete endoprosthesis for a hip joint, with a femur head on a shaft (1), of which the core (12) below the femur head comprises a curved upper section flattened in a directin perpendicular to a medial/lateral plane, and a conically tapering lower section, with a nose-like lug (14) arranged on the lateral face of the upper shaft section and extending in a medial/lateral plane, characterised in that the nose-like lug (14) is provided on its lateral end section remote from the shaft (1) with a transversely extending flange (16).

2. Femural complete endoprosthesis according to claim 1, characterised in that the flange (16) is tapered downwardly.

3. Femural complete endoprosthesis according to claim 1 or 2, characterised in that the flange (16), at least on its outside (17), is made curved towards the shaft (1).

4. Femural complete endoprosthesis according to claim 3, characterised in that the curved flange (16) is aligned with the lateral surface of the lower shaft section of the shaft core (12).

5. Femural complete endoprosthesis according to one of the preceding claims, characterised in that the flange (16) is made symmetrically of the lug (14) on both sides.

6. Femural complete endoprosthesis according to one of the preceding claims, characterised in that the nose-like lug (14) and/or the flange (16) is provided with passage openings (19).

EP 0 238 860 B1

FIG. 1

FIG.2

FIG.3

FIG.4

FIG.5